# EUROPEAN PATENT APPLICATION

(11) **EP 4 006 129 A1**
(43) Date of publication of application: **01.06.2022**
(21) Application number: 20853154.1
(22) Date of filing: 29.05.2020
(51) Int. Cl.: C11B 9/00, A61L 9/01

(54) **AROMATIC DEODORANT AND AROMATIC DEODORIZATION DEVICE**

(30) Priority: 09.08.2019 JP 2019147551
(71) Applicant: Daikin Industries, Ltd., Osaka-shi, Osaka 530-8323 (JP)
(72) Inventor: MIKI Sanae, Osaka-shi, Osaka 530-8323 (JP); TAKESHITA Goushi, Osaka-shi, Osaka 530-8323 (JP); AKIYAMA Ryuuji, Osaka-shi, Osaka 530-8323 (JP); KOYAMA Chika, Osaka-shi, Osaka 530-8323 (JP)
(74) Representative: Goddar, Heinz J.
(86) International application number: PCT/JP2020/021446
(87) International publication number: WO 2021/029123

(57) **Abstract**

An aromatic deodorant (C) removes a target malodor containing n-valeric acid. The aromatic deodorant (C) contains at least limonene, citral, citronellal, and cinnamaldehyde.

## Description

### TECHNICAL FIELD

The present disclosure relates to an aromatic deodorant and an aromatic deodorization device.

### BACKGROUND ART

Sensory deodorization has been known as a method for removing malodors. This sensory deodorization uses scent components to prevent malodors from being sensorily perceived. Patent Document 1 discloses a scent composition capable of sensorily removing aging body odor.

### CITATION LIST

### PATENT DOCUMENT

Patent Document 1: Japanese Unexamined Patent Publication No. H11-286428

### SUMMARY

### TECHNICAL PROBLEM

In the sensory deodorization, mixing of malodors and scent components may cause people to feel unpleasant depending on the types, concentrations, combination, and other factors of the malodors and the scent components. Thus, in some cases, the sensory deodorization provides no deodorant effect.

Here, human body odors, sweat odors, and sebum odors contain n-valeric acid. The n-valeric acid has a smell that tends to cause people to feel unpleasant even in a small amount. Even if an attempt is made to add a scent component to a target malodor containing the n-valeric acid to sensorily remove the target malodor, people feel unpleasant in some cases.

It is an object of the present disclosure to sensorily remove a target malodor containing n-valeric acid.

### SOLUTION TO THE PROBLEM

A first aspect of the present disclosure is directed to an aromatic deodorant (C) for removing a target malodor containing n-valeric acid. The aromatic deodorant (C) contains: limonene; citral; citronellal; and cinnamaldehyde.

According to the first aspect, adding at least limonene, citral, citronellal, and cinnamaldehyde to the target malodor containing n-valeric acid enables sensory deodorization.

A second aspect of the present disclosure is an embodiment of the first aspect. In the second aspect, the target malodor is a care-environment odor.

Odors in a care environment, i.e., so-called care-environment odors, contain n-valeric acid. In the second aspect, care-environment odors can be sensorily removed.

A third aspect of the present disclosure is an embodiment of the first or second aspect. In the third aspect, the target malodor contains acetaldehyde.

Acetaldehyde is a substance that may cause people to feel unpleasant after addition of a specific scent component thereto. In the third aspect, the target malodor containing acetaldehyde can also be sensorily removed.

A fourth aspect of the present disclosure is directed to an aromatic deodorization device (10) for delivering a deodorant component for removing a target malodor containing n-valeric acid. The aromatic deodorization device (10) contains: an aromatic deodorant (C) that is a source of the deodorant component. The aromatic deodorant (C) is the aromatic deodorant of any one of the first to third aspects.

### BRIEF DESCRIPTION OF THE DRAWINGS

FIG. 1 is a schematic diagram of an overall configuration of a target space that contains an aromatic deodorization device according to a first embodiment.
FIG. 2 is a schematic diagram of a configuration of the aromatic deodorization device.
FIG. 3 is a block diagram of the aromatic deodorization device.
FIG. 4 is a table showing samples for use in an evaluation test 1 for a deodorant effect.
FIG. 5 is a table showing evaluation criteria of a nine-level pleasantness/unpleasantness indication method for use in the evaluation test for the deodorant effect.
FIG. 6 is a table showing evaluation criteria of a six-level odor intensity indication method for use in the evaluation test for the deodorant effect.
FIG. 7 is a graph showing change in the pleasantness/unpleasantness level between the case where the odor intensity of only n-valeric acid was evaluated and the case where the intensity of each mixture of odors obtained by adding a first scent to n-valeric acid was evaluated.
FIG. 8 is a table showing samples for use in an evaluation test 2 for a deodorant effect.
FIG. 9 is a table showing the results of the evaluation test 2 obtained when the average odor intensity of a sample B 1 is 2.10.
FIG. 10 corresponds to FIG. 9, and shows the results obtained when the average odor intensity of the sample B1 is 3.60.
FIG. 11 corresponds to FIG. 7, and shows results obtained when the odor intensity of only n-valeric acid was evaluated and when the intensity of each mixture of odors obtained by adding a second scent to n-valeric acid was evaluated.
FIG. 12 corresponds to FIG. 7, and shows the results obtained when the odor intensity of only acetaldehyde was evaluated and when the intensity of each mixture of odors obtained by adding the second scent to acetaldehyde was evaluated.
FIG. 13 is a table showing the results of an evaluation test 5 obtained when the average odor intensity of a sample E1 is 3.14.
FIG. 14 corresponds to FIG. 13, and shows the results obtained when the average odor intensity of the sample E1 is 3.29.
FIG. 15 is a table showing a combination of components of a target malodor for use in an evaluation test 6 for a deodorant effect.
FIG. 16 is a table showing the results of the evaluation test 6 obtained when the average odor intensity of a sample F1 is 2.90.
FIG. 17 corresponds to FIG. 9, and shows the results obtained when the average odor intensity of the sample F1 is 3.05.

### DESCRIPTION OF EMBODIMENTS

### «First Embodiment»

A first embodiment will be described.

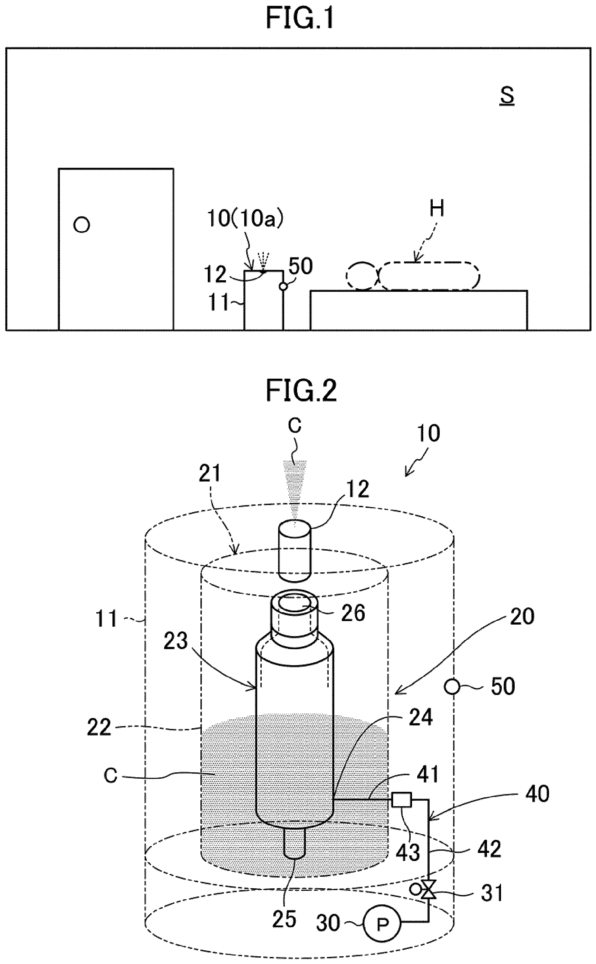

As shown in FIG. 1, an aromatic deodorization device (10) of the present disclosure is placed in an indoor space (S) of an elderly care home, a hospital, or any other similar facility. A person (H) who emits a care-environment odor as a target malodor is present in the indoor space (S). In the example of FIG. 1, the person (H) is the source of the malodors. The care-environment odor as used herein means a malodor emitted from aged sebum, aged urine, or any other substance associated with the life of an elderly person, a patient, or a handicapped person. The care-environment odor contains n-valeric acid and acetaldehyde.

The aromatic deodorization device (10) of the present disclosure sprays an aromatic deodorant (C) into the indoor space (S), and generates a deodorant component. The aromatic deodorant (C) serves as a source of a predetermined deodorant component. The aromatic deodorant (C) removes the target malodor through the deodorant component contained in the aromatic deodorant. The aromatic deodorant (C) is a scent. The aromatic deodorant (C) contains scent components.

The scent components contained in the aromatic deodorant (C) of this embodiment consist of limonene, citral, citronellal, and cinnamaldehyde. In other words, the scent components of the aromatic deodorant (C) are four components such as limonene, citral, citronellal, and cinnamaldehyde. The contents of the scent components in the aromatic deodorant (C) may be selected as appropriate depending on different components from the foregoing components, the deodorant effect for removing the target malodor, and other factors, but are not specifically limited.

The aromatic deodorant (C) sprayed by the aromatic deodorization device (10) enables sensory deodorization. The phrase "sensory deodorization" as used herein includes "masking" and "modulation." In the "masking," the sensitivity to the malodors is reduced by a relatively strong smell of a scent component. In the "modulation," the malodor is mixed with a scent component to change the quality of a smell affecting a person's sense of smell, and thus makes it difficult for the person to perceive the smell as a malodor. The aromatic deodorization device (10) of this embodiment sprays a relatively small amount of the aromatic deodorant (C) according to the malodor to modulate the malodor.

### -Aromatic Deodorization Device-

As illustrated in FIG. 2, the aromatic deodorization device (10) includes a sprayer body (10a), and a sensor (50) integrally attached to the sprayer body (10a). The sprayer body (10a) includes a casing (11), a spray unit (20) housed inside the casing (11) and a control unit (60).

### <Casing>

The casing (11) is hollow. The casing (11) has a supply port (12) through which the atomized aromatic deodorant (C) is supplied into the indoor space (S).

### <Spray Unit>

The spray unit (20) includes a spray cartridge (21), an air pump (30), an air flow path (40), and an electromagnetic valve (31).

The spray cartridge (21) is detachably attached to the inside of the casing (11). The spray cartridge (21) includes a tank (22) and a two-fluid nozzle (23). The tank (22) stores the aromatic deodorant (C). The two-fluid nozzle (23) constitutes an atomizing mechanism that atomizes the aromatic deodorant (C) in the tank (22). Specifically, the two-fluid nozzle (23) atomizes a liquid membrane of the aromatic deodorant (C) by the shearing force of an airflow.

The two-fluid nozzle (23) has an inflow port (24) through which air flows thereinto, a suction port (25) through which the aromatic deodorant (C) is sucked thereinto, and a spray port (26) through which the atomized aromatic deodorant (C) is discharged. The inflow port (24) is formed in the peripheral wall of the body of the two-fluid nozzle (23). The suction port (25) is formed at the lower end of the body of the two-fluid nozzle (23). The spray port (26) is formed at the upper end of the body of the two-fluid nozzle (23).

The air pump (30) has a discharger connected to the air flow path (40). The air pump (30) sucks the air in the indoor space (S) and discharges the sucked air to the air flow path (40). The air pump (30) supplies the air to the two-fluid nozzle (23) of the spray cartridge (21).

The air flow path (40) includes an inner flow path (41) and an outer flow path (42). The inner flow path (41) is located inside the spray cartridge (21). The outer flow path (42) is located outside the spray cartridge (21). The inner flow path (41) and the outer flow path (42) are connected to each other via a fluid joint (43). The fluid joint (43) is located outside the spray cartridge (21). The inner flow path (41) has an outflow end connected to the inflow port (24) of the two-fluid nozzle (23). The outer flow path (42) has an inflow end connected to the air pump (30).

The electromagnetic valve (31) is connected to the outer flow path (42). The electromagnetic valve (31) is an on-off valve that opens and closes the air flow path (40).

### <Sensor>

The sensor (50) detects an index indicating the odor strength of a malodor (in this embodiment, a care-environment odor) generated in the indoor space (S). The "index indicating the odor strength of the malodor" as used herein includes the concentration of a substance with the malodor, the odor concentration of the malodor, and the odor intensity proportional to the concentration of the substance with the malodor. The sensor (50) of this embodiment detects the odor intensity of a malodor corresponding to a care-environment odor.

The sensor (50) of this embodiment is integral with the sprayer body (10a) (see FIGS. 1 and 2). Strictly speaking, the sensor (50) is provided on the casing (11) so as to be exposed to the indoor space (S).

### <Control Unit>

The control unit (60) of this embodiment controls the spray amount of the aromatic deodorant (C) sprayed from the spray unit (20) in accordance with a value detected by the sensor (50). The control unit (60) includes a microcomputer on a control board and a memory device (specifically, a semiconductor memory) storing software for operating the microcomputer.

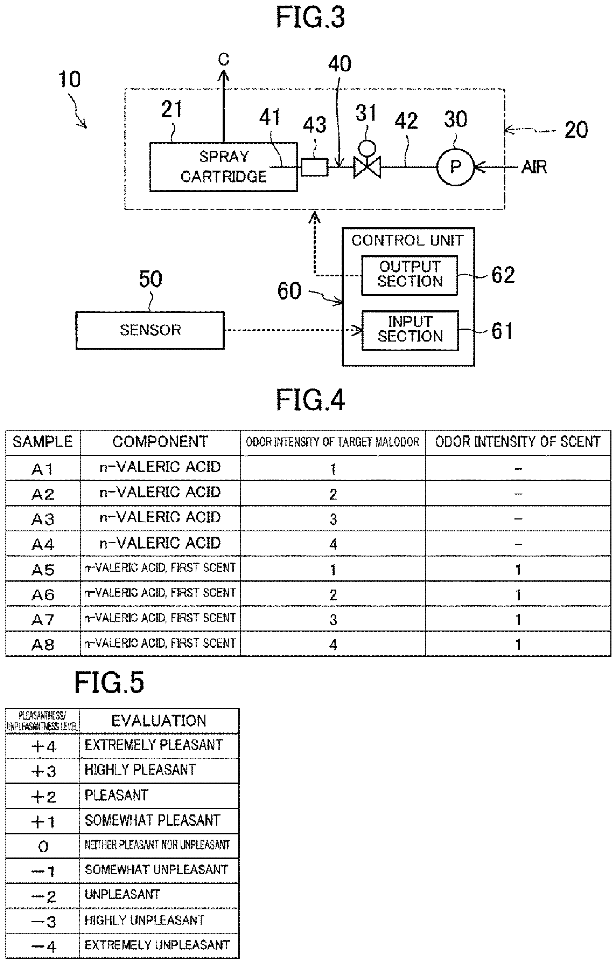

As shown in FIG. 3, the control unit (60) includes an input section (61) and an output section (62). The input section (61) receives a signal indicating the odor intensity of a malodor detected by the sensor (50). The output section (62) outputs a control signal intended to satisfy the spray amount corresponding to the signal received by the input section (61) to the spray unit (20).

The spray amount is adjusted by adjusting the time interval between intermittent operations of the air pump (30), the time period during which the electromagnetic valve (31) is opened/closed, the flow rate of the air discharged by the air pump (30), the opening degree of the air flow path (40), and other parameters.

### -Operation-

An operation of the aromatic deodorization device (10) will be described.

When a malodor is generated in the indoor space (S), a person in the space feels unpleasant. After the generation of the malodor, the sensor (50) detects the odor intensity of the malodor, which is then fed to the input section (61) of the control unit (60). When the input section (61) receives the detected value, the output section (62) of the control unit (60) outputs the control signal to the spray unit (20). This enables a spraying operation of the aromatic deodorization device (10).

In the spraying operation, the air pump (30) is turned on, and the electromagnetic valve (31) is open. The air transferred by the air pump (30) is introduced into the spray cartridge (21). The two-fluid nozzle (23) atomizes the aromatic deodorant (C) by the flow of this air. The atomized aromatic deodorant (C) is supplied through the spray port (26) and the supply port (12) into the indoor space (S).

Starting the spraying operation in the foregoing manner allows the deodorant component delivered from the aromatic deodorant (C) to provide an adequate modulation effect from immediately after the generation of the malodor. This can reduce the level of unpleasantness of the person in the space.

### -Sensory Deodorant Effect of Aromatic Deodorant According to First Embodiment-

Evaluation results of a sensory deodorant effect of the aromatic deodorant according to the first embodiment (hereinafter referred to also as a first scent) will be described.

### (1) Scent Components of Aromatic Deodorant

The scent components contained in the first scent for use in evaluation tests 1 and 2 consist of limonene, citral, citronellal, and cinnamaldehyde.

### (2) Evaluation Test 1 for Deodorant Effect

In this test, samples were first put into respective bags, and subjects then smelled an odor in each of the bags. Next, the subjects sensorily evaluated the pleasantness/unpleasantness level of the odor in each bag.

The target malodor of this test is n-valeric acid. The samples of this test are samples A1 to A8 of respective eight odors as shown in FIG. 4. Specifically, the samples A1 to A4 are each only of the target malodor. The samples A1, A2, A3, and A4 have odor intensities adjusted to 1, 2, 3, and 4, respectively. The samples A5 to A8 are mixtures of odors obtained by adding the first scent that has an odor intensity adjusted to 1 to the samples A1 to A4.

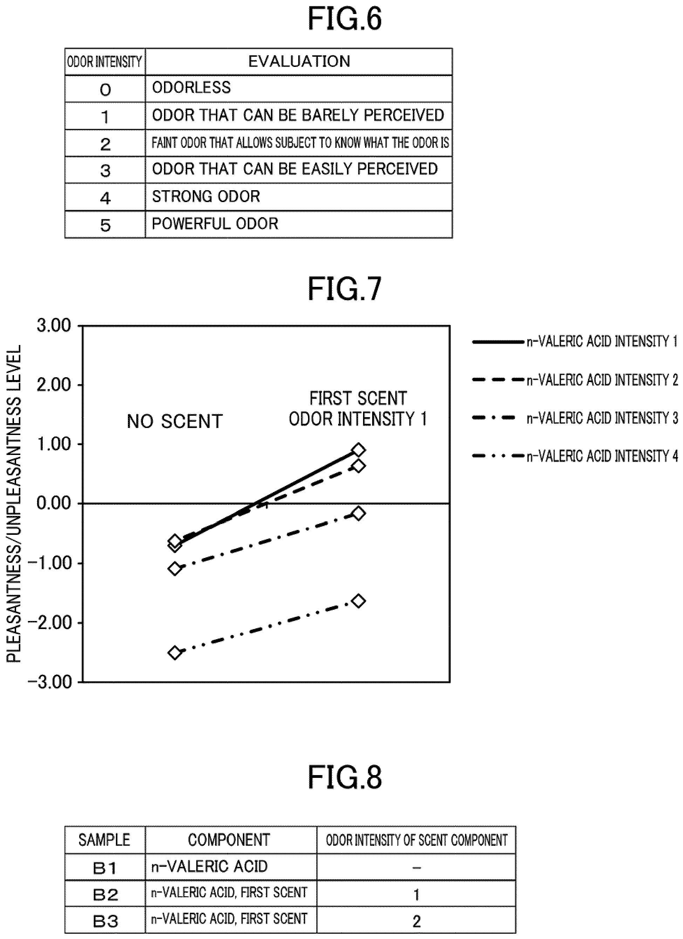

The number of subjects is 15. The subjects evaluated the pleasantness/unpleasantness level based on criteria of a nine-level pleasantness/unpleasantness indication method shown in FIG. 5. Then, the levels evaluated by the subjects were averaged. Note that the odor intensities of the target malodor and the scent were adjusted using a six-level odor intensity indication method shown in FIG. 6.

The evaluation results were compared between the samples that are equal in the odor intensity of the target malodor. As shown in FIG. 7, in each of the cases where the odor intensity of the target malodor was 1 to 4, the pleasantness/unpleasantness level of the mixture of odors obtained by adding the first scent to the target malodor (the samples A5 to A8) was higher than that of only the malodor (the sample A1 to A4). This demonstrated that irrespective of the odor intensity of n-valeric acid as the target malodor, the first scent allows the odor of n-valeric acid to be sensorily removed.

### (3) Evaluation Test 2 for Deodorant Effect

A test method and an evaluation indexes in this test are similar to those in the evaluation test 1.

The target malodor of this test is n-valeric acid. Samples of this test are samples B1 to B3 that of respective three odors as shown in FIG. 8. Specifically, the sample B1 is only of the target malodor that has an odor intensity unadjusted. The sample B2 is a mixture of odors of the sample B1 and the first scent that has an odor intensity adjusted to 1. The sample B3 is a mixture of odors of the sample B1 and the scent that has an odor intensity adjusted to 2. The number of subjects is 15. In this test, the subjects sensorily evaluated the odor intensity of the sample B1 as well.

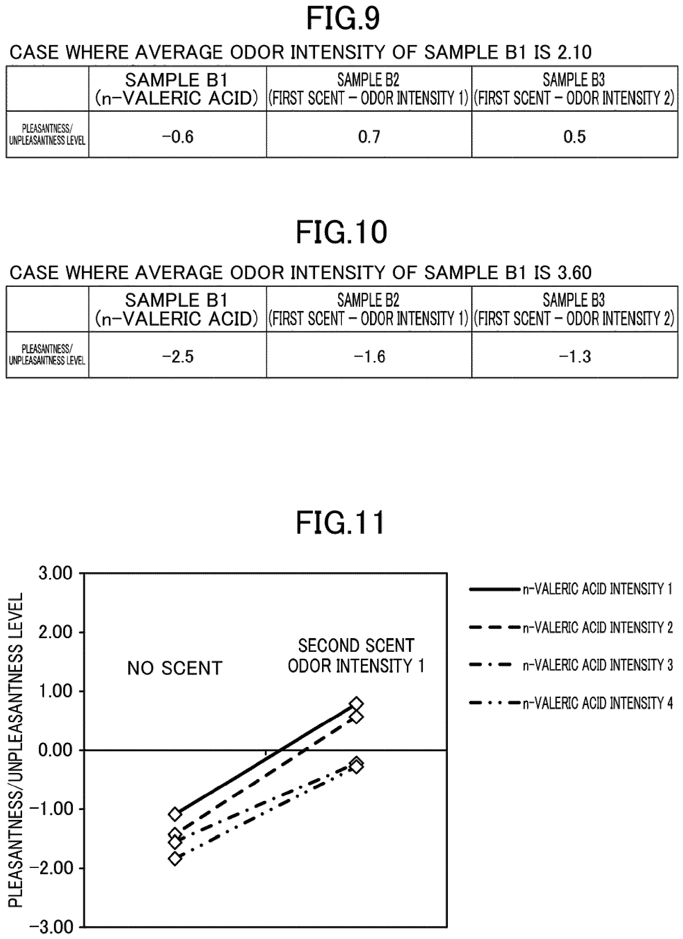

The results of this test are shown in FIGS. 9 and 10. FIG. 9 shows the results obtained when the average odor intensity of the sample B1 is 2.10. FIG. 10 shows the results obtained when the average odor intensity of the sample B1 is 3.60.

Increasing the odor intensity of the scent added to the target malodor would not always result in an increase in the pleasantness/unpleasantness level of the target malodor. According to the results of this test, the pleasantness/unpleasantness levels of the samples B2 and B3 were higher than the pleasantness/unpleasantness level of the sample B1. This demonstrated that adding the first scent with different odor intensities to n-valeric acid which has the target malodor also enabled sensory deodorization.

### -Feature (1) of First Embodiment-

The aromatic deodorant (C) of this embodiment removes the target malodor containing n-valeric acid. This aromatic deodorant (C) contains at least limonene, citral, citronellal and cinnamaldehyde.

In this embodiment, adding at least limonene, citral, citronellal, and cinnamaldehyde to the target malodor containing n-valeric acid enables sensory deodorization.

### -Feature (2) of First Embodiment-

The target malodor of this embodiment is a care-environment odor.

Odors in a care environment, i.e., so-called care-environment odors, contain n-valeric acid. The aromatic deodorant (C) of this embodiment can sensorily remove care-environment odors.

### -Feature (3) of First Embodiment-

The aromatic deodorization device (10) of this embodiment delivers the deodorant component for removing the target malodor containing n-valeric acid. This aromatic deodorization device (10) includes the aromatic deodorant (C) that is as a source of the deodorant components.

### «Second Embodiment»

A second embodiment will be described.

An aromatic deodorization device (10) of this embodiment is obtained by replacing the aromatic deodorant (C) stored in the tank (22) of the spray cartridge (21) in the aromatic deodorization device (10) of the first embodiment. An aromatic deodorant (C) of this embodiment will now be described.

### -Aromatic Deodorant-

Scent components contained in the aromatic deodorant (C) of this embodiment consist of limonene, citral, citronellal, cinnamaldehyde, carvone, trans-2-hexenal, benzyl benzoate, and benzaldehyde. In other words, the scent components of the aromatic deodorant (C) are eight components of limonene, citral, citronellal, cinnamaldehyde, carvone, trans-2-hexenal, benzyl benzoate, and benzaldehyde.

### -Sensory Deodorant Effect of Aromatic Deodorant According to Second Embodiment-

Evaluation results of a sensory deodorant effect of the aromatic deodorant according to the second embodiment (hereinafter referred to also as a second scent) will be described.

### (1) Scent Components of Aromatic Deodorant

The scent components contained in the second scent for use in the evaluation tests 3 to 6 consist of limonene, citral, citronellal, cinnamaldehyde, carvone, trans-2-hexenal, benzyl benzoate, and benzaldehyde. Note that the second scent contains all of the scent components contained in the first scent.

### (2) Evaluation Test 3 for Deodorant Effect

A test method and an evaluation indexes in this test are similar to those in the evaluation test 1. The target malodor of this test is n-valeric acid. Samples of this test are samples C1 to C8 that each contain the second scent instead of the scent added in the evaluation test 1. The number of subjects is 15. The evaluation results were compared between the samples that are equal in the odor intensity of the target malodor.

As shown in FIG. 11, in each of the cases where the odor intensity of the target malodor was 1 to 4, the pleasantness/unpleasantness level of the mixture of odors obtained by adding the second scent to the target malodor (the samples C5 to C8) was higher than that of only the malodor (the sample C1 to C4). This demonstrated that irrespective of the odor intensity of n-valeric acid as the target malodor, the second scent enabled sensory deodorization.

### (3) Evaluation Test 4 for Deodorant Effect

A test method and an evaluation indexes in this test are similar to those in the evaluation test 1. The target malodor of this test is acetaldehyde. Samples of this test are samples D1 to D8 that each contain acetaldehyde instead of the target malodor of the evaluation test 3. The number of subjects is 15. The evaluation results were compared between the samples that are equal in the odor intensity of the target malodor.

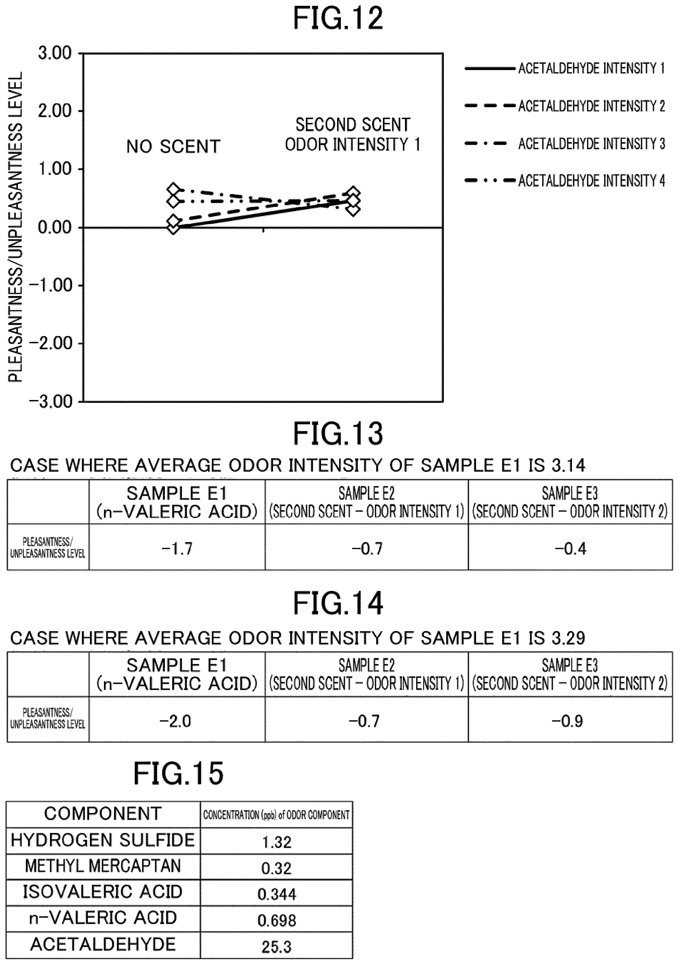

Acetaldehyde is a substance that may cause people to feel unpleasant after addition of a specific scent component thereto. However, as shown in FIG. 12, in each of the cases where the odor intensity of the target malodor was 1 to 4, no significant change was observed between the pleasantness/unpleasantness level of only the malodor (the sample D1 to D4) and the pleasantness/unpleasantness level of the mixture of odors obtained by adding the second scent to the target malodor (the samples D5 to D8). This demonstrated that irrespective of the odor intensity of acetaldehyde as the target malodor, the second scent added to the target malodor less affected the sensory deodorant effect. In addition, since the second scent contains all of the scent components of the first scent, it can be estimated that the aromatic deodorant (C) of the first embodiment also provides similar advantages.

### (4) Evaluation Test 5 for Deodorant Effect

A test method and an evaluation indexes in this test are similar to those in the evaluation test 1. The target malodor of this test is n-valeric acid. Samples of this test are samples E1 to E3 that each contain the second scent instead of the scent added in the evaluation test 2. The number of subjects is seven. In this test, the subjects sensorily evaluated the odor intensity of the sample E1 as well.

The results of this test are shown in FIGS. 13 and 14. FIG. 13 shows the results obtained when the average odor intensity of the sample E1 is 3.14. FIG. 14 shows the results obtained when the average odor intensity of the sample E1 is 3.29.

Increasing the odor intensity of the scent added to the target malodor would not always result in an increase in the pleasantness/unpleasantness level of the target malodor. According to the results of this test, the pleasantness/unpleasantness levels of the samples E2 and E3 were higher than the pleasantness/unpleasantness level of the sample E1. This demonstrated that adding the second scent with different odor intensities to n-valeric acid as the target malodor also enabled sensory deodorization.

### (5) Evaluation Test 6 for Deodorant Effect

A test method and an evaluation indexes in this test are similar to those in the evaluation test 1. The target malodor of this test is a complex odor prepared by mixing the components at the mixing ratio shown in FIG. 15. This complex odor was obtained by the mixing to simulate an odor in a care environment. Samples of this test are samples F1 to F3 that each contain the complex odor instead of the target malodor of the evaluation test 5. The number of subjects is 20. In this test, the subjects sensorily evaluated the odor intensity of the sample F1 as well.

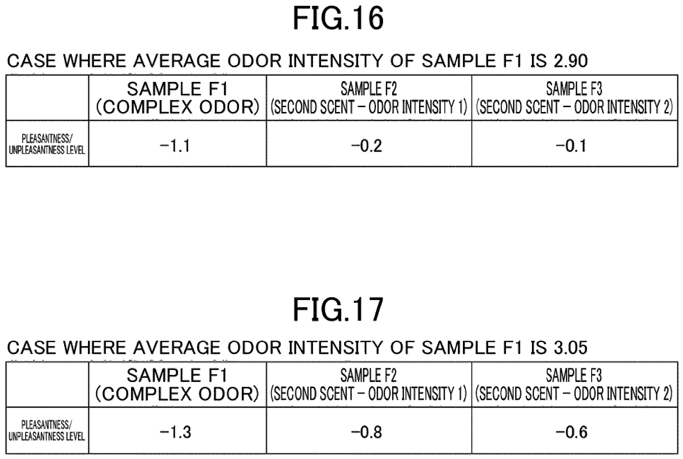

The results of this test are shown in FIGS. 16 and 17. FIG. 16 shows the results obtained when the average odor intensity of the sample F1 is 2.90. FIG. 17 shows the results obtained when the average odor intensity of the sample F1 is 3.05.

According to the results of this test, the pleasantness/unpleasantness levels of the samples F2 and F3 were higher than the pleasantness/unpleasantness level of the sample F1. This demonstrated that adding the second scent with different odor intensities to the complex odor as the target malodor also enabled sensory deodorization.

### -Feature (1) of Second Embodiment-

The target malodor of this embodiment contains acetaldehyde.

Acetaldehyde is a substance that may cause people to feel unpleasant after addition of a specific scent component thereto. The aromatic deodorant (C) of this embodiment can sensorily remove the target malodor containing acetaldehyde as well.

### «Other Embodiments»

The above embodiment may also be configured as follows.

The target malodor in each of the foregoing embodiments merely needs to contain n-valeric acid, but may be an odor other than the care-environment odors. Examples of the target malodor include a shoe-rack odor and a pet odor.

The aromatic deodorant (C) of each of the foregoing embodiments may contain a diluent, such as ethanol and water.

The aromatic deodorization device (10) of each of the foregoing embodiments does not have to include the sensor (50).

The atomizing mechanism of the aromatic deodorization device (10) of each of the forging embodiments may be of another type, such as a piezoelectric type, an electrostatic spray type, or an ultrasonic type.

The aromatic deodorization device (10) of each of the foregoing embodiments includes the electromagnetic valve (31), but may instead include a flow rate control valve capable of finely adjusting the opening degree of the air flow path (40).

A plurality of spray units (20) may be provided for the aromatic deodorization device (10) of each of the foregoing embodiments.

The aromatic deodorization device (10) of each of the foregoing embodiments may be used for an air cleaner, an air conditioner, a ventilator, a humidifier, a dehumidifier, and any other device.

The aromatic deodorant (C) of each of the foregoing embodiments may deliver deodorant components by using a different system, such as a spray system, besides the aromatic deodorization device (10) of a mechanical spray system similar to that of each of the foregoing embodiments.

While the embodiments and variations thereof have been described above, it will be understood that various changes in form and details may be made without departing from the spirit and scope of the claims. The foregoing embodiments and variations thereof may be combined and replaced with each other without deteriorating the intended functions of the present disclosure.

### INDUSTRIAL APPLICABILITY

As can be seen from the foregoing description, the present disclosure is useful for an aromatic deodorant and an aromatic deodorization device.

### DESCRIPTION OF REFERENCE CHARACTERS

- C: Aromatic Deodorant
- 10: Aromatic Deodorization Device
- 20: Spray Unit
- 30: Air Pump
- 31: Electromagnetic Valve
- 40: Air Flow Path
- 50: Sensor
- 60: Control Unit

## Claims

1. An aromatic deodorant (C) for removing a target malodor containing n-valeric acid, the aromatic deodorant (C) comprising:
limonene; citral; citronellal; and cinnamaldehyde.

2. The aromatic deodorant of claim 1, wherein
the target malodor is a care-environment odor.

3. The aromatic deodorant of claim 1 or 2, wherein
the target malodor contains acetaldehyde.

4. An aromatic deodorization device (10) for delivering a deodorant component for removing a target malodor containing n-valeric acid, the aromatic deodorization device (10) comprising:
an aromatic deodorant (C) that is a source of the deodorant component,
the aromatic deodorant (C) being the aromatic deodorant of any one of claims 1 to 3.
